(19) Europäisches Patentamt
European Patent Office
Office européen des brevets

(11) Publication number : **0 276 998 B1**

# (12) EUROPEAN PATENT SPECIFICATION

(45) Date of publication of patent specification : **02.05.91 Bulletin 91/18**

(51) Int. Cl.$^5$ : **C07D 319/06**, A61K 7/46

(21) Application number : **88300689.2**

(22) Date of filing : **27.01.88**

## (54) Perfumery materials.

(30) Priority : **29.01.87 GB 8701961**

(43) Date of publication of application : **03.08.88 Bulletin 88/31**

(45) Publication of the grant of the patent : **02.05.91 Bulletin 91/18**

(84) Designated Contracting States : **AT BE CH DE ES FR GB GR IT LI NL SE**

(56) References cited :
DE-A- 2 526 675
GB-A- 2 073 175
US-A- 4 220 593
CHEMICAL ABSTRACTS, vol. 72, 1970, no. 131393q, Columbus, Ohio, US; A.V. BOGATSKII et al.: "Antimicrobial and fungicidal activity of alkoxyalkyl-substituted 1,3-dioxanes" & FISIOL. AKTIV. VESHCHESTVA 1969, no. 2, 124-8

(73) Proprietor : **UNILEVER PLC**
**Unilever House Blackfriars P.O. Box 68**
**London EC4P 4BQ (GB)**
**GB**
Proprietor : **UNILEVER NV**
**Burgemeester s'Jacobplein 1 P.O. Box 760**
**NL-3000 DK Rotterdam (NL)**
**BE CH DE ES FR GR IT LI NL SE AT**

(72) Inventor : **Newman, Christopher Paul**
**5 The Foreland Nackington Road**
**Canterbury Kent CT1 3NT (GB)**
Inventor : **Rossiter, Karen Jane**
**1 Park Place Beaver Road**
**South Ashford Kent TN23 1SL (GB)**
Inventor : **Sell, Charles Stanley**
**Parsonage Farm Church Lane**
**Aldington Kent TN25 7EG (GB)**

(74) Representative : **Dries, Antonius Johannes Maria et al**
**Unilever N.V. Patent Division P.O. Box 137**
**NL-3130 AC Vlaardingen (NL)**

**EP 0 276 998 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Field of the invention :

This invention relates to novel perfumery materials having a cyclic acetal structure and organoleptic properties. The novel materials within this class are of value in the preparation of perfume compositions of use in fabric washing formulations, toilet soaps and toilet preparations and air fresheners, as examples.

Background to the invention :

There is a general requirement in perfumery for component materials providing a variety of perfume notes in a composition. The perfumery materials within the class described herein provide notes over a wide range of odours, in particular certain members of the series possess a surprising amber odour.

General description of the invention :

The present invention provides a class of perfumery compounds of the general formula I

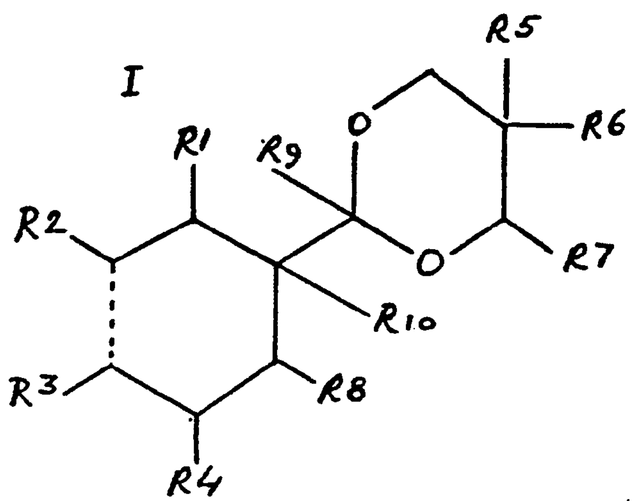

where the dotted line denotes a double or single bond within the cyclohexane ring and wherein R1, R2, R3 and R4 are each hydrogen or methyl R5 and R6 are each hydrogen, or a (cyclo) alkyl or (cyclo) alkenyl group containing 1 to 4 carbon atoms or an optionally substituted ring containing up to 5 carbon atoms or, together, form an optionally substituted ring containing up to 6 carbon atoms, including the carbon atom in the dioxane ring ; R7 is hydrogen, methyl, ethyl, n-propyl, isopropyl, n-butyl, sec.butyl, isobutyl, tert.butyl ; R8 and R9 are each hydrogen or methyl ; R10 is hydrogen, methyl or isopropyl, with the proviso that the total number of C atoms is in the range 13 to 20, preferably 15 or more carbon atoms.

Examples of the groups defined by R5 and R6 are methyl, ethyl, propyl, isopropyl, n-butyl, isobutyl, tert.butyl, sec.butyl, vinyl, propenyl, isopropenyl, butenyl, isobutenyl, sec.butenyl, cyclopropyl cyclo butyl and, together, an optionally substituted cyclohex(en) yl ring.

These perfumery compounds are usable as odiferous components in perfume compositions. The amount of perfumery compound present will be dependent on the proposed use of the composition and will usually be at least about 0.1% by weight. The composition may contain up to about 10% or up to 95% by weight of at least one of the perfumery compounds described.

Preferred chemical compounds have R1, R2, R3, R4, R8 and R9 as defined above, R5 and R6 are each hydrogen, methyl, ethyl, sec.butyl, tert.butyl, n-propyl, isopropyl or a substituted hex-3-ene ring, R7 is hydrogen or n-propyl and R10 is hydrogen or methyl.

In this general formula no stereochemistry is implied. However it has been observed that odour character varies amongst the stereochemical isomers of some of the products of this invention. For example, products with the amber note generally demonstrate a stronger amber character in the more volatile stereoisomers. This effect is believed to be related to cis/trans isomerism about the dioxane ring. These isomers were separated for chemical and organo-leptic evaluation as pairs of diasteromers by glc and/or fractional distillation.

The perfumery compositions of the present invention are detectable and appreciated by the olfactory sense.

Such compositions may be used as commercial products as they stand, eg. handkerchief perfumes, aftershaves and toilet waters or added to specific products, eg soaps, detergents, shampoos, skin creams, talcum powders, polishes and products releasing perfume into a space.

These cyclic acetals and ketals are conveniently formed by reacting a 1,3-diol of formula II with an aldehyde or ketone of formula III

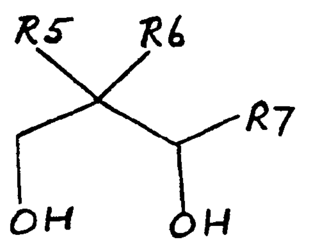 II 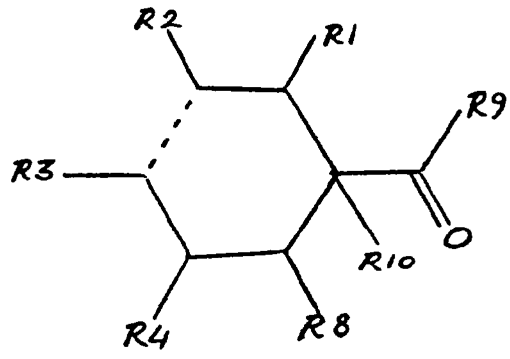 III

wherein the groups R1 to R10 inclusive are as defined in claim 1.

The 1,3-diol is preferably selected from the group
2-methyl-2-sec.butylpropane-1,3-diol,
2-methyl-2-propylpropane-1,3-diol, 2-ethylhexane-1,
3-diol, 2-methyl-2-isopropylpropane-1,3-diol and
2-methyl-2-tert.butylpropane-1,3-diol.

The diols utilised in the preparation of the claimed compounds are obtainable from commercial sources or prepared using well characterised techniques. Thus the diols 2-methyl-2-isopropylpropane-1,3-diol ;
2-methyl-2-tert.butyl-propane-1,3-diol and
2-methyl-2-(1-methylpropyl)propane-1,3-diol are prepared respectively from 3-methylbutanal, 3,3-dimethylbutanal and 3-methylpentanal by the following process. The appropriate aldehyde is subjected to a Mannich reaction with formaldehyde and diethylamine to introduce an alpha-methylene group. The alkene bond in the product is hydrogenated at atmospheric pressure over 5% palladium on carbon. The saturated aldehyde so formed is treated with two equivalents of formaldehyde in the presence of a base. This gives the desired diol in a combined aldol condensation and Cannizzaro reaction.

## Literature

GB 1549213 describes cyclic acetals and ketals used as inflammation inhibitors in cosmetic preparations. A number of acetals and ketal are listed on page 2, line 37 et seq. of that patent specification. In Chem. Abstr. 70 (1970), no.131393q some cyclic acetals are described as having antimicrobial and fungicidal activity. More specifically the following compounds falling within the definition of formula I are disclosed in these two references :
compounds wherein R1-R4 and R8 and R10 are all hydrogen and wherein

i) R5 and R6 are methyl if R7 is isopropyl or propyl and R9 is hydrogen, or

ii) R5 and R6 are methyl if R7 is isopropyl and R9 is methyl, or

iii) R5 and R7 and R9 are hydrogen if R6 is propyl or methyl, or

iv) R5 and R6 are ethyl if R7 and R9 are hydrogen ; compounds wherein R2, R3, R4, R8, R9, and R10 are hydrogen and R1, R5, R6 and R7 are all methyl ; compounds wherein R1, R2, R4, R5, R6, R9 and R10 are hydrogen and R3, R7 and R8 are methyl.

## Specific description of the invention :

Examples of the perfumery materials of the present invention and a process for their preparation will now be given to illustrate the invention.

## Example I

This example describes the preparation of the perfumery material which is a mixture of compounds in which

i) R1, R3, R7, R8, R9 and R10 are hydrogen, R2, R4 and R5 are methyl and R6 is sec.butyl and ii) R2, R4, R7, R8, R9 and R10 are hydrogen ; R1, R3, R5 are methyl and R6 is sec.butyl, and the dotted line represents a double bond in both cases.

A 3-necked round bottom flask (250 ml capacity) was fitted with a mechanical stirrer, thermometer and Dean and Stark apparatus, the latter was equipped with a double surface water condenser. Privetal (0.1 mole, 14.6 g) and 2-methyl-2-sec.butylpropane-1,3 diol (0.1 mole 14.6 g) were charged into the flask together with cyclohexane (50 ml) as solvent and a catalytic amount of p-toluenesulphonic acid (0.35 g). Privetal was a mixture of 2,4-dimethyl-and 3,5-dimethyl-cyclohex-3-ene-1-carboxaldehyde obtained from PPF International, Ashford, England ; now obtained under the trade name Ligustral from Quest International, Ashford, Kent the successor company to PPF International. (The 1,3-diol was obtained from Hoechst GmbH of West Germany.)

The reaction mixture was heated under reflux at 90°C and atmospheric pressure for 2.5 hours. At the end of this period no more water was removed as an azeotrope with the cyclohexane solvent. After cooling the mixture to ambient temperature, anhydrous sodium carbonate (0.5 g) was added to neutralise and dry the solution while stirring was continued for 1 hour. The mixture was then filtered and the solvent removed by evaporation at 30°C under water pump vacuum. A 72% yield of crude product was obtained. The crude material was distilled using a 50 ml flask equipped with a Vigreux column (6.0 × 1.5 cm), a nitrogen bleed and a thermometer. The distillation was performed at a pressure of 6 mm of mercury and the desired mixture of acetals was collected over the temperature range of 135-136°C. The yield of pure product was 50%.

GCMS showed the product to consist of several isomers with a relative molecular mass of 266. The cyclic acetal structures were supported by IR and proton NMR spectra.

This perfumery component has an intense and very persistent ambery character with woody or floral lily-like overtones.

The compounds prepared were :

2-(3,5-dimethyl-3-cyclohexen-1-yl)-5-methyl-5-(1-methylpropyl)-1,3-dioxane and

2-(2,4-dimethyl-3-cyclohexen-1-yl)-5-methyl-5-(1-methylpropyl)-1,3-dioxane.

Example II

Example I was repeated using Privetal and 2-methyl-2-propylpropane-1,3-diol (obtained from Hoechst GmbH of West Germany).

The yield of the pure product, consisting of the isomers in which R1, R3, R7, R8, R9 and R10 are hydrogen, R2, R4 and R5 are methyl and R6 n-propyl, and R2, R4, R7, R8, R9, R10 are hydrogen, R1, R3, R5 are methyl and R6 is n-propyl and the dotted line in both cases represents a double bond was 80%. The product distilled over the temperature range 127°C to 128°C at 5 mm mercury.

This material possesses a novel and long-lasting green note with amber, sandalwood and earthy nuances.

The compounds prepared were :

2-(3,5-dimethyl-3-cyclohexen-1-yl)-5-methyl-5-propyl-1,3-dioxane and

2-(2,4-dimethyl-3-cyclohexen-1-yl)-5-methyl-5-propyl-1,3-dioxane.

Example III

Example I was repeated using Isocyclocitral (a mixture of isomers of 2,4,6-trimethyl- and 3,5,6-trimethyl-cyclohex-3-ene-1-carboxaldehyde) as substitute for Privetal. Isocyclocitral is obtained from Quest International. The product was identical in structure to those of Example I except R8 is methyl and not hydrogen. The yield was 46%, the product distilled over the temperature range 135°C to 140°C at 0.5 mm mercury. This perfume material possesses a citrus/sulphurous note.

The compounds prepared were :

2-(3,5,6-trimethyl-3-cyclohexen-1-yl)-5-methyl-5-(1-methylpropyl)-1,3-dioxane and

2-(2,4,6-trimethyl-3-cyclohexen-1-yl)-5-methyl-5-(1-methylpropyl)-1,3-dioxane.

Example IV

Example III was repeated using 2-methyl-2-propyl propane-1,3-diol and Isocyclocitral. The product was identical in structure to that obtained in Example II except R8 is methyl and not hydrogen. The yield was 71%, the product distilled over the temperature range 146°C to 147°C at 2 mm mercury. This perfume material has a note comparable with that of the Example II product material but has a lower intensity.

The compounds prepared were :

2-(3,5,6,-trimethyl-3-cyclohexen-1-yl)-5-methyl-5-propyl-1,3-dioxane and

2-(2,4,6-trimethyl-3-cyclohexen-1-yl)-5-methyl-5-propyl-1,3-dioxane.

Example V

Example I was repeated using 3-cyclohexene-1-carboxaldehyde (obtained from Degussa GmbH of West Germany) as equimolar substitute for Privetal. The product has R1, R2, R3, R4, R7, R8, R9 and R10 hydrogen, R5 methyl and R6 sec.butyl and the dotted line represents a double bond. The yield was 51%, the product distilled over the temperature range 134°C to 138°C at 1 mm mercury. This perfume material possesses a bitter grapefruit peel note.

The compound prepared was :
2-(3-cyclohexen-1-yl)-5-methyl-5-(1-methylpropyl)-1, 3-dioxane.

Example VI

The procedure of example I was repeated using equimolar quantities of Privetal and 2-ethyl hexane-1,3-diol (obtained from Hoechst GmbH). The product was a mixture i) R1, R3, R5, R8, R9 and R10 are hydrogen, R2 and R4 are methyl ; R6 is ethyl and R7 is n-propyl ii) R2, R4, R5, R8, R9 and R10 are hydrogen ; R1 and R3 are methyl ; R6 is ethyl and R7 is n-propyl ; in both isomers the dotted line is a double bond. The yield was 77%, the product distilled over the temperature range 138°C to 142°C at 4.5 mm mercury.

The material possesses a soft, clean, fresh, citrus peel note.
The compounds prepared were :
2-(3,5-dimethyl-3-cyclohexen-1-yl)-5-ethyl-4-propyl-1 ,3-dioxane and
2-(2,4-dimethyl-3-cyclohexen-1-yl)-5-ethyl-4-propyl-1,3-dioxane.

Example VII

The procedure of example I was repeated using equimolar quantities of 1,3,5-trimethyl- and 1,2, 4-trimethyl-cyclohex-3-ene-1-carboxaldehyde (prepared by Diels-Alder reaction of 2-methyl-1,3-pentadiene and methacrolein) and 2-methyl-2-sec.butylpropane-1,3-diol. The product was a mixture of isomers i) R1, R3, R7, R8 and R9 are hydrogen ; R2, R4, R5 and R10 are methyl ; R6 is sec.butyl and ii) R2, R4, R7, R8 and R9 are hydrogen ; R1, R3, R5 and R10 are methyl ; R6 is sec.butyl ; in both isomers the dotted line is a double bond. The yield was 43%, the product distilled at 150°C at 3.5 mm mercury.

The material possesses a smooth, weak, dry woody, ambery note.
The compounds prepared were :
2-(1,3,5-trimethyl-3-cyclohexen-1-yl)-5-methyl-5-(1-methylpropyl)-1,3-dioxane and
2-(1,2,4-trimethyl-3-cyclohexen-1-yl)-5-methyl-5-(1 methylpropyl)-1,3-dioxane.

Example VIII

The procedure of example I was repeated using equimolar quantities of a mixture of 1-acetyl-3, 5-dimethylcyclohex-3-ene and the 2,4-dimethyl isomer (prepared by Diels-Alder reaction of 2-methyl-1,3-pentadiene and methyl vinyl ketone) together with 2-methyl-2-sec.butylpropane-1,3-diol. The product was a mixture of isomers i) R1, R3, R7, R8 and R10 are hydrogen ; R2, R4, R5 and R9 are methyl and R6 is sec. butyl and ii) R2, R4, R7, R8 and R10 are hydrogen, R1, R3, R5 and R9 are methyl and R6 is sec.butyl ; the dotted line is a double bond in both isomers. The yield was 61%, the product distilled over the temperature range 142°C to 144°C at 2 mm mercury.

The material possesses a sweet, honey, orange blossom note with light green undertones.
The compounds prepared were :
2-(3,5-dimethyl-3-cyclohexen-1-yl)-2,5-dimethyl-5-(1methylpropyl)-1,3-dioxane and
2-(2,4-dimethyl-3-cyclohexen-1-yl)-2,5-dimethyl-5 (1-methylpropyl)-1,3-dioxane.

Example IX

The procedure of example VIII was repeated but using 2-methyl-2-propylpropane-1,3-diol as the diol reactant. The product was a mixture of isomers i) R1, R3, R7, R8 and R10 are hydrogen ; R2, R4, R5 and R9 are methyl and R6 is n-propyl and ii) R2, R4, R7, R8 and R10 are hydrogen ; R1, R3, R5 and R9 are methyl and R6 is n-propyl ; in both isomers the dotted line is a double bond. The yield was 30%, the product distilled over the temperature range 120°C to 121°C at 4 mm mercury.

The material posseses a weak pleasant orris, orange-blossom note.

The compounds prepared were :

2-(3,5-dimethyl-3-cyclohexen-1-yl)-2,5-dimethyl-5-propyl-1,3-dioxane and

2-(2,4-dimethyl-3-cyclohexen-1-yl)-2,5-dimethyl-5-propyl-1,3-dioxane.

Example X

The procedure of example VIII was repeated but using 2-ethylhexane-1,3-diol as the diol reactant. The product was a mixture of isomers i) R1, R3, R5, R8 and R10 are hydrogen ; R2, R4 and R9 are methyl, R6 is ethyl and R7 is n-propyl and ii) R2, R4, R5, R8 and R10 are hydrogen ; R1, R3 and R9 are methyl ; R6 is ethyl and R7 is n-propyl ; in both isomers the dotted line is a double bond. The yield was 17%, the product distilled over the temperature range 90°C to 95°C at 0.1 mm mercury.

The material possesses a weak, lactone, musky odour.

The compounds prepared were :

2-(3,5-dimethyl-3-cyclohexen-1-yl)-5-ethyl-2-methyl-4-propyl-1,3-dioxane and

2-(2,4-dimethyl-3-cyclohexen-1-yl)-5-ethyl-2-methyl-4-propyl-1,3-dioxane.

Example XI

The procedure of example I was repeated using equimolar quantities of a mixture of 1,3,5- and 1,2,4-trimethyl-cyclohex-3-ene-1-carboxaldehyde (prepared as in Example 8) and 2-ethylhexane-1,3-diol. The product was a mixture in which i) R1, R3, R5, R8 and R9 are hydrogen, R2, R4 and R10 are methyl, R6 is ethyl and R7 is n-propyl and ii) R2, R4, R5, R8 and R9 are hydrogen ; R1, R3, and R10 are methyl ; R6 is ethyl and R7 is n-propyl, in both isomers the dotted line is a double bond. The yield was 39%, the product distilled over the temperature range 100°C to 105°C at 0.1 mm mercury.

The material possesses a weak, fruity odour, reminiscent of apple, passionfruit and peach.

The compounds prepared were :

2-(1,3,5-trimethyl-3-cyclohexen-1-yl)-5-ethyl-4-propyl-1,3-dioxane and

2-(1,2,4-trimethyl-3-cyclohexen-1-yl)-5-ethyl-4-propyl-1,3-dioxane.

Example XII

The procedure of example III was repeated using 2-ethylhexane-1,3-diol as the diol reactant. The product was identical in structure to that obtained in example VII except R8 is methyl and not hydrogen. The yield was 48%, the product distilled over the temperature range 130°C to 135°C at 6 mm mercury.

The material possessed an interesting myrrh note.

The compounds prepared were :

2-(3,5,6-trimethyl-3-cyclohexen-1-yl)-5-ethyl-4-propyl-1,3-dioxane and

2-(2,4,6-trimethyl-3-cyclohexen-1-yl)-5-ethyl-4-propyl-1,3-dioxane.

Example XIII

This example describes the preparation of the perfumery material which is a mixture of compounds in which i) R1, R3, R7, R8, R9 and R10 are hydrogen ; R2, R4 and R5 are methyl and R6 is sec.butyl and ii) R2, R4, R7, R8, R9 and R10 are hydrogen ; R1, R3 and R5 are methyl and R6 is sec.butyl, and the dotted line denotes a single bond in both cases.

A 3-necked round bottom flask (100 ml capacity) was fitted with a mechanical stirrer, thermometer, gas inlet tube and gas outlet tube via a water condenser. The material as prepared in example I (0.05 mole, 13.3 g) was charged into the flask together with cyclohexane (40 ml) as solvent and a catalytic amount of platinum oxide (0.3 g $PtO_2$ + water, 83% Pt, ex. Hopkin and Williams Ltd). The mixture was stirred vigorously and freed from dissolved oxygen by bubbling nitrogen through for fifteen minutes. The flow of nitrogen was then stopped and the system purged using hydrogen for 5 minutes. The system was shut off from the atmosphere and hydrogen was still admitted into the system using a gas meter to measure the volume of hydrogen consumed. The temperature rose from 20°C to 35°C during the first 25 minutes then fell slowly. After ca. 4 hours, uptake of hydrogen had ceased, a total of 1000 ml having been consumed. The supply of hydrogen was then shut off and the system purged by bubbling nitrogen through. The catalyst was removed by filtration under gravity and the solvent removed by evaporation at 30°C under water pump vacuum. The crude material was distilled at a pressure of 0.1 mm mercury. The desired mixture of acetals was collected at 90°C. The yield of pure product

was 46%.

GCMS showed the product to consist of several isomers with a relative molecular mass of 268. The cyclic acetal structures were supported by IR and proton NMR spectra.

The material possessed a green and ambery note reminscent of lily of the valley.

The compounds prepared were :

2-(3,5-dimethylcyclohexyl)-5-methyl-5-(1-methyl-propyl)-1,3-dioxane and

2-(2,4-dimethylcyclohexyl)-5-methyl-5-(1-methyl propyl)-1,3-dioxane.

Example XIV

The hydrogenation procedure of example XIII was repeated but using the product obtained from Example II. The product of hydrogenation was a mixture in which i) R1, R3, R7, R8, R9 and R10 are hydrogen, R2, R4 and R5 are methyl and R6 is n-propyl and ii) R2, R4, R7, R8, R9 and R10 are hydrogen, R1, R3 and R5 are methyl and R6 is n-propyl with the dotted line denoting a single bond in both compounds. The yield was 73%, the product distilled at 90°C and a pressure of 0.1 mm mercury.

The materials posesses a weak woody note. The compounds were :

2-(3,5-dimethylcyclohexyl)-5-methyl-5-propyl-1,3-dioxane

2-(2,4-dimethylcyclohexyl)-5-methyl-5-propyl-1,3-dioxane.

Example XV

Example I was repeated using Privetal and 2-methyl-2-tert.butylpropane-1,3-diol. The product was a mixture of isomers i) R1, R3, R7, R8, R9 and R10 are hydrogen ; R2, R4 and R5 are methyl and R6 is tert-butyl and ii) R2, R4, R7, R8, R9 and R10 are hydrogen ; R1, R3 and R5 are methyl and R6 is tert-butyl and the dotted line both cases represents a double bond. The yield was 57%, the product distilled at 110°C and 0.2 mm mercury.

This material posseses an amber, woody character with almond top notes.

The compounds prepared were :

2-(3,5-dimethyl-3-cyclohexen-1-yl)-5-methyl-5-tert-butyl-1,3-dioxane and

2-(2,4-dimethyl-3-cyclohexen-1-yl)-5-methyl-5-tert-butyl-1,3-dioxane.

Example XVI

Example I was repeated using Privetal and 2-methyl-2-isopropylpropane-1,3-diol. The product was a mixture of i) R1, R3, R7, R8, R9 and R10 are hydrogen ; R2, R4 and R5 and methyl and R6 is isopropyl and ii) R2, R4, R7, R8, R9 and R10 are hydrogen ; R1, R3 and R5 are methyl and R6 is isopropyl and the dotted line in both cases represents a double bond. The yield was 25%, the product distilled over the temperature range 90°C to 95°C at 0.2 mm mercury.

This material possesses fresh green, sappy and amber notes.

The compounds prepared were :

2-(3,5-dimethyl-3-cyclohexen-1-yl)-5-methyl-5-isopropyl-1,3-dioxane and

2-(2,4-dimethyl-3-cyclohexen-1-yl)-5-methyl-5-isopropyl-1,3-dioxane.

EXAMPLE XVII

Example I was repeated using a mixture of 1,1 bis (hydroxymethyl)-3,5-dimethylcyclohex-3-ene and 1,1 bis (hydroxymethyl)-2,4-dimethylcyclohex-3-ene (obtained by combined Aldol and Canizzaro rection of Privetal with two equivalents of formaldehyde in the presence of a base as described for the acyclic diols) as an equimolar substitute for the diol.

The yield of product, consisting of isomers in which :

i) R1, R3, R7, R8, R9 and R10 are hydrogen, R2, R4 are methyl and R5 and R6 together with the carbon of the dioxane ring to which they are attached form a 2,4-dimethylcyclohex-3-ene ring

ii) R2, R4, R7, R8, R9 and R10 are hydrogen, R1, R3 are methyl and R5 and R6 together (as above) form a 2,4-dimethyl cyclohex-3-ene ring

iii) R1, R3, R7, R8, R9 and R10 are hydrogen, R2, R4 are methyl and R5 and R6 together (as above) form a 3,5-dimethylcylohex-3-ene ring

iv) R2, R4, R7, R8, R9 and R10 are hydrogen, R1, R3 are methyl and R5 and R6 together (as above) form a 3,5-dimethycyclohex-3-ene ring and the dotted line in all cases represented a double bond, was 52%.

The product was purified on a Leybold Heraeus wiped film evaporator with a jacket temperature of 95-100°C and internal pressure of 0.01 mm Hg.
This material posseses fruity and slightly herbal notes.
The compounds prepared were :
7,9-dimethyl-3-($2^1$,$4^1$-dimethyl-$3^1$-cyclohexen-$1^1$-yl)-2,4-dioxaspiro (5.5) undec-8-ene.
8,10-dimethyl-3-($2^1$,$4^1$-dimethyl-$3^1$-cyclohexen-$1^1$-yl)-2,4-dioxaspiro (5.5) undec-8-ene.
7,9-dimethyl-3-($3^1$,$5^1$-dimethyl-$3^1$-cyclohexen-$1^1$-yl)-2,4-dioxaspiro (5.5) undec-8-ene.
8,10-dimethyl-3-($3^15^1$-dimethyl-$3^1$-cyclohexen-$1^1$-yl)-2,4-dioxaspiro (5.5) undec-8-ene.

## Claims

1. A perfume composition containing, as an odiferous component, an effective amount of at least one perfumery compound of the general formula I

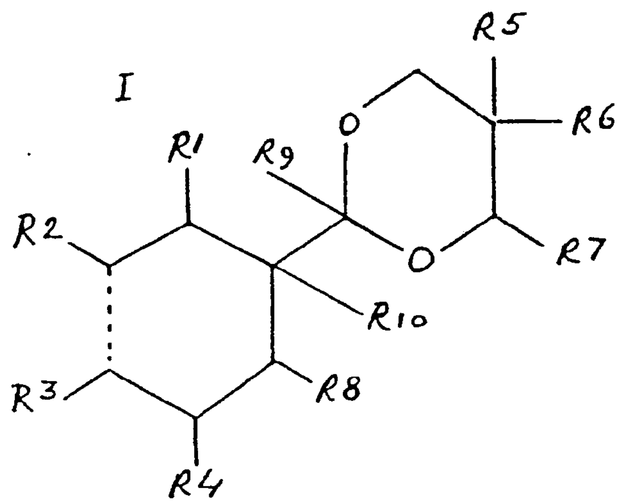

where the dotted line denotes a double or single bond within the cyclohexane ring and wherein R1, R2, R3 and R4 are each hydrogen or methyl ; R5 and R6 are each hydrogen, or an alkyl, alkenyl, cycloalkyl or cycloalkenyl group containing 1 to 4 carbon atoms or, together, form an optionally substituted ring containing up to 6 carbon atoms, including the carbon atom in the dioxane ring ; R7 is hydrogen, methyl, ethyl, n-propyl, isopropyl, n-butyl, sec.butyl, isobutyl, tert.butyl ; R8 and R9 are each hydrogen or methyl ; R10 is hydrogen, methyl or isopropyl, with the proviso that the total number of C atoms is in the range 13 to 20.

2. A perfume composition according to claim 1 containing at least about 0.1% by weight of at least one perfumery compound defined in claim 1.

3. A perfume composition according to claim 1 or 2 containing up to about 95% by weight of at least one perfumery compound defined in claim 1.

4. 2-(3,5-dimethyl-3-cyclohexen-1-yl)-5-methyl-5-tert.butyl-1,3-dioxane

5. 2-(2,4-dimethyl-3-cyclohexen-1-yl)-5-methyl-5-tert.butyl-1,3-dioxane.

6. 2-(3,5-dimethyl-3-cyclohexen-1-yl)-5-methyl-5-isopropyl-1,3-dioxane.

7. 2-(2,4-dimethyl-3-cyclohexen-1-yl)-5-methyl-5-isopropyl-1,3-dioxane.

8. 2-(3,5-dimethyl-3-cyclohexen-1-yl)-5-methyl-5-(1-methylpropyl)-1,3-dioxane.

9. 2-(2,4-dimethyl-3-cyclohexen-1-yl)-5-methyl-5-(1-methylpropyl)-1,3-dioxane.

10. 2-(3,5-dimethyl-3-cyclohexen-1-yl)-5-methyl-5-propyl-1,3-dioxane.

11. 2-(2,4-dimethyl-3-cyclohexen-1-yl)-5-methyl-5propyl-1,3-dioxane.

12. 2-(2,4-dimethylcyclohexyl)-5-methyl-5-(1-methylpropyl)-1,3-dioxane.

13. 2-(3,5-dimethylcyclohexyl)-5-methyl-5-(1-methylpropyl)-1,3-dioxane.

14. The use of compounds of claims 4 to 13 in perfumery compositions.

## Ansprüche

1. Duftzusammensetzung, die als geruchtragende Komponente eine wirksame Menge mindestens einer

Parfümverbindung der allgemeinen Formel I

I

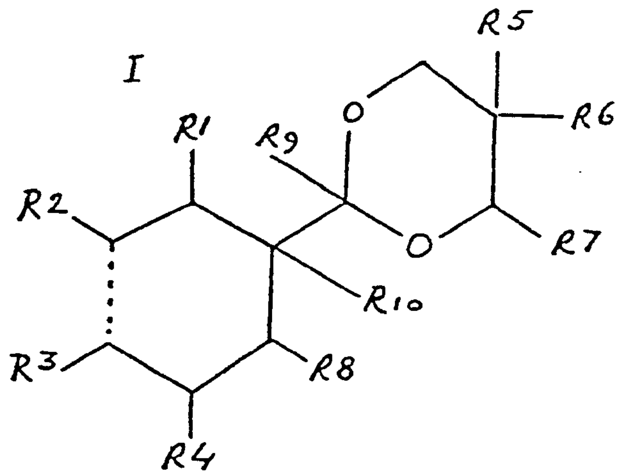

enthält, in welcher die gestrichelte Linie eine Doppel- oder Einfachbindung innerhalb des Cyclohexanringes anzeigt und $R^1$, $R^2$, $R^3$ und $R^4$ jeweils Wasserstoff oder Methyl bedeuten, $R^5$ und $R^6$ jeweils Wasserstoff oder eine Alkyl-, Alkenyl-, Cycloalkyl- oder Cycloalkenylgruppe mit 1 bis 4 Kohlenstoffatomen bedeuten oder gemeinsam einen gegebenenfalls substituierten Ring bilden, der bis zu 6 Kohlenstoffatome, einschließlich des Kohlenstoffatoms im Dioxanring,enthält, $R^7$ Wasserstoff, Methyl, Ethyl, n-Propyl, Isopropyl, n-Butyl-, s-Butyl, Isobutyl, t-Butyl bedeutet, $R^8$ und $R^9$ jeweils Wasserstoff oder Methyl bedeuten, $R^{10}$ Wasserstoff, Methyl oder Isopropyl bedeutet, mit der Maßgabe, daß die Gesamtanzahl der Kohlenstoffatome zwischen 13 und 20 liegt.

2. Duftzusammensetzung gemäß Anspruch 1, die mindestens etwa 0,1 Gew.-% mindestens einer in Anspruch 1 definierten Parfümverbindung enthält.

3. Duftzusammensetzung gemäß Anspruch 1 oder 2, die bis zu etwa 95 Gew.-% mindestens einer in Anspruch 1 definierten Parfümverbindung enthält.

4. 2-(3,5-Dimethyl-3-cyclohexen-1-yl)-5-methyl-5-t-butyl-1,3-dioxan.

5. 2-(2,4-Dimethyl-3-cyclohexen-1-yl)-5-methyl-5-t-butyl-1,3-dioxan.

6. 2-(3,5-Dimethyl-3-cyclohexen-1-yl)-5-methyl-5-isopropyl-1,3-dioxan.

7. 2-(2,4-Dimethyl-3-cyclohexen-1-yl)-5-methyl-5-isopropyl-1,3-dioxan.

8. 2-(3,5-Dimethyl-3-cyclohexen-1-yl)-5-methyl-5-(1-methylpropyl)-1,3-dioxan.

9. 2-(2,4-Dimethyl-3-cyclohexen-1-yl)-5-methyl-5-(1-methylpropyl)-1,3-dioxan.

10. 2-(3,5-Dimethyl-3-cyclohexen-1-yl)-5-methyl-5-propyl-1,3-dioxan.

11. 2-(2,4-Dimethyl-3-cyclohexen-1-yl)-5-methyl-5-propyl-1,3-dioxan.

12. 2-(2,4-Dimethylcyclohexyl)-5-methyl-5-(1-methylpropyl)-1,3-dioxan.

13. 2-(3,5-Dimethylcyclohexyl)-5-methyl-5-(1-methylpropyl)-1,3-dioxan.

14. Die Verwendung der Verbindungen gemäß den Ansprüchen 4 bis 13 in Duftzusammensetzungen.

## Revendications

1. Composition de parfum qui contient, à titre de composant odoriférant, une quantité efficace d'au moins un composé de parfumerie de formule générale I

I

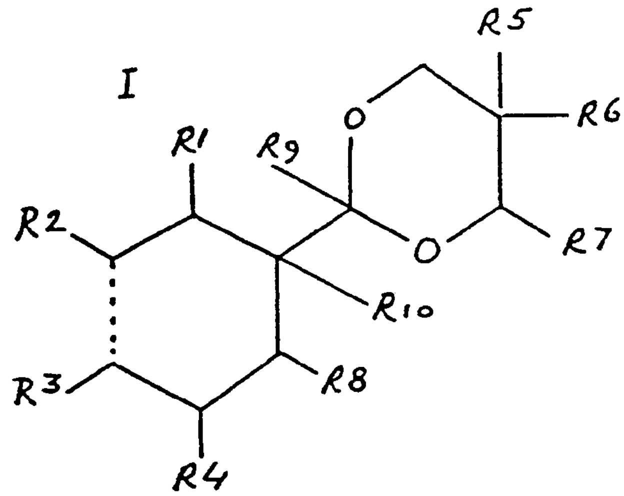

dans laquelle la ligne en pointillés représente une double liaison ou une simple liaison dans le noyau cyclohexane et dans laquelle R1, R2, R3 et R4 sont chacun un atome d'hydrogène ou le groupe méthyle ; R5 et R6 sont chacun un atome d'hydrogène ou un groupe alkyle, alcényle, cycloalkyle ou cycloalcényle contenant 1 à 4 atomes de carbone, ou conjointement forment un noyau éventuellement substitué contenant jusqu'à 6 atomes de carbone y compris l'atome de carbone dans le noyau dioxanne ; R7 est un atome d'hydrogène, un groupe méthyle, éthyle, n-propyle, isopropyle, n-butyle, sec-butyle, isobutyle, tert-butyle ; R8 et R9 sont chacun un atome d'hydrogène ou le groupe méthyle ; R10 est un atome d'hydrogène, le groupe méthyle ou isopropyle, à la condition que le nombre total d'atomes de carbone soit compris dans l'intervalle allant de 13 à 20.

2. Composition de parfum selon la revendication 1, qui contient au moins environ 0,1% en poids d'au moins un composé de parfumerie défini dans la revendication 1.

3. Composition de parfum selon la revendication 1 ou 2 qui contient jusqu'à environ 95% en poids d'au moins un composé de parfumerie défini dans la revendication 1.

4. 2-(3,5-diméthyl-3-cyclohexèn-1-yl)-5-méthyl-5-tert.butyl-1,3-dioxanne.

5. 2-(2,4-diméthyl-3-cyclohexèn-1-yl)-5-méthyl-5-tert.butyl-1,3-dioxanne.

6. 2-(3,5-diméthyl-3-cyclohexèn-1-yl)-5-méthyl-5-isopropyl-1,3-dioxanne.

7. 2-(2,4-diméthyl-3-cyclohexèn-1-yl)-5-méthyl-5-isopropyl-1,3-dioxanne.

8. 2-(3,5-diméthyl-3-cyclohexèn-1-yl)-5-méthyl-5-(1-méthylpropyl)-1,3-dioxanne.

9. 2-(2,4-diméthyl-3-cyclohexèn-1-yl)-5-méthyl-5-(1-méthylpropyl)-1,3-dioxanne.

10. 2-(3,5-diméthyl-3-cyclohexèn-1-yl)-5-méthyl-5-propyl-1,3-dioxanne.

11. 2-(2,4-diméthyl-3-cyclohexèn-1-yl)-5-méthyl-5-propyl-1,3-dioxanne.

12. 2-(2,4-diméthylcyclohexyl)-5-méthyl-5-(1-méthylpropyl)-1,3-dioxanne.

13. 2-(3,5-diméthylcyclohexyl)-5-méthyl-5-(1-méthylpropyl)-1,3-dioxanne.

14. Utilisation des composés selon les revendications 4 à 13 dans des compositions de parfumerie.